# EUROPEAN PATENT SPECIFICATION

(11) **EP 0 684 805 B1**
(45) Date of publication and mention of the grant of the patent: **20.06.2001**
(21) Application number: 94906342.4
(22) Date of filing: 16.02.1994
(51) Int. Cl.: A61F 5/01

(54) **LIMB PROTECTOR**
ARM- ODER BEINSCHUTZ
ELEMENT DE PROTECTION DE MEMBRE

(30) Priority: 17.02.1993 GB 9303147
(43) Date of publication of application: 06.12.1995
(73) Proprietor: Doyle, Kelvin, Conrad, Harrow Middlesex HA3 6QJ (GB)
(72) Inventor: Doyle, Kelvin, Conrad, Harrow Middlesex HA3 6QJ (GB)
(74) Representative: Enskat, Michael Antony Frank
(86) International application number: GB9400318
(87) International publication number: WO9418916

(56) References cited:
- WO-A-91/01701
- DE-A- 3 905 837
- US-A- 4 139 002
- US-A- 4 554 913
- US-A- 4 884 561
- US-A- 5 086 760

## Description

This invention relates to a limb protector, particularly a protector for a jointed limb, that is a knee or elbow protector.

Injuries to limbs and to joints occur for several reasons, including participation in contact sports such as American football or ice hockey, in individual sports such as skiing or motor cycle racing, or indeed any active sport. In addition, injuries to limbs and joints occur in active occupations such as the police or armed forces, or in any type of accident.

After an injury occurs, the limb or joint affected by a minor injury is conventionally supported by elastic supports which give slight restriction of movement or by rigid splints for major injuries, giving complete immobilization.

In some cases, preventative equipment if available. Hinged knee braces (de-rotational braces) are available to prevent twisting of a knee, and can be worn during activities such as skiing, but by their nature are restrictive, the hinges permit only forward bending. Braces are available for sports such as American football which protect the knee against side impact, or frontal impact, but also restrict movement, (prophylactic or preventive braces). Dynamic braces are also available, with eccentrically placed hinges to provide protection to torn ligaments.

In more extreme form, riot police can wear body armour. In a different sphere, the medical profession use light weight splints to give complete immobilization of a knee or elbow when rotational movement is likely to cause damage, especially for use in accidents to permit safe transport of the person to hospital.

In many cases, a small amount of movement of the affected joint might be beneficial to healing, and indeed speed recovery, but existing equipment allows either relatively large twisting movements, as with elastic supports, or no twisting movement, as with hinged braces.

In the application by Fratesi, WO 91/01701, separate knee and thigh guards are connected by a hinge at the knee joint, but the wearer's knee can move only by bending in the vertical plane.

In Offenlegungschnift DE 3905837, K and K Inc, a three-part knee protector is hinged at upper and lower hinging points, but the wearer's knee can move only by bending in the vertical plane.

In US Patent No 4,884,561, a knee brace is hinged at a single pivot point, and the wearer's knee can move only by bending in the vertical plane.

The current invention provides a broader range of facilities than any existing brace, and can be used in prophylactic, dynamic, or de-rotational mode.

According to the invention a limb protector comprises an upper rigid curved support; a lower rigid curved support; and means for permitting relative rotation of the upper support and the lower support, such relative movement taking place in a plane substantially parallel to the planes of curvature of the upper and lower supports. Preferably the amount of relative rotation is adjustable. Preferably there is also provided means for permitting relative hinging of the upper and lower supports in a plane orthogonal to the plane of relative rotation.

In use the upper rigid curved support is placed around the upper part of a limb, such as a thigh or upper arm, and the lower rigid curved support is placed around the lower part of a limb, such as a lower leg or forearm. The means permitting relative hinge of the supports permits the knee or elbow to bend in a plane which in the case of the knee will be substantially vertical with the relative rotation being in a substantially horizontal plane, permitting the knee to twist, to a degree which can be preset.

Preferably, either the upper or the lower rigid curved support is provided in two-part form with a sliding joint between the parts so as to permit relative rotation of the upper and lower supports. In the case of a knee protector, the sliding joint is provided in the lower support adjacent to the upper support, preferably arranged so that relative rotation occurs immediately below the knee.

Preferably there is also provided a rigid curved direct impact protector which overlaps the lower portion of the upper support and the upper portion of the lower support. The direct impact protector protects a knee joint from front impact and an elbow joint from rear impact.

The impact protector also acts as a hinge cover by overlapping the adjacent edges of the upper and lower supports during a hinging movement. The cover may be in two parts, pivotally connected to each other and one part being pivotally connected to each of the upper and lower supports.

optionally there is further provided at least one rigid side impact protector, overlapping the lower portion of the upper support and the upper portion of the lower support and positioned to protect a joint within the supports from side impact.

Preferably the rigid material of which the curved supports are made is resistant material such as carbon-fibre reinforced plastics material.

The invention will now be described by way of example only with reference to the accompanying drawings in which:
Figure 1 is an exploded view of a knee protector according to the invention;
Figure 2 is a detailed view of part of Figure 1 illustrating the relative rotation of the upper and lower limb supports at the front of the support;
Figure 3 is a detailed view of part of Figure 2;
Figure 4 is a detailed view of a further part of Figure 1 illustrating the relative rotation of the upper and lower limb supports at the rear of the support;
Figure 5 is a view of an assembled, alternative embodiment of a knee protector according to the invention;
Figure 6 is an example of an adjustable fastening mechanism for a limb protector;
Figure 7 is an example of means for adjusting the size of a knee protector for width fitting;
Figure 8 illustrates means for allowing hinging movement;
Figure 9 is an example of a wall material;
Figure 10 illustrates the knee protection of Figure 1 in assembled form;
Figure 11 illustrates how hinging action allows the protector to be put on a human knee;
Figure 12 illustrates a variation of the protector of Figure 1;
Figure 13 illustrates the protector of Figure 1 from in front and from the rear;
Figure 14 is a variation of the protector illustrated in Figure 1;
Figure 15 is a view of the inside of the Figure 14 embodiment;
Figure 16 shows the assembly of the Figure 1 variation;
Figure 17 illustrates a variation of Figure 14.

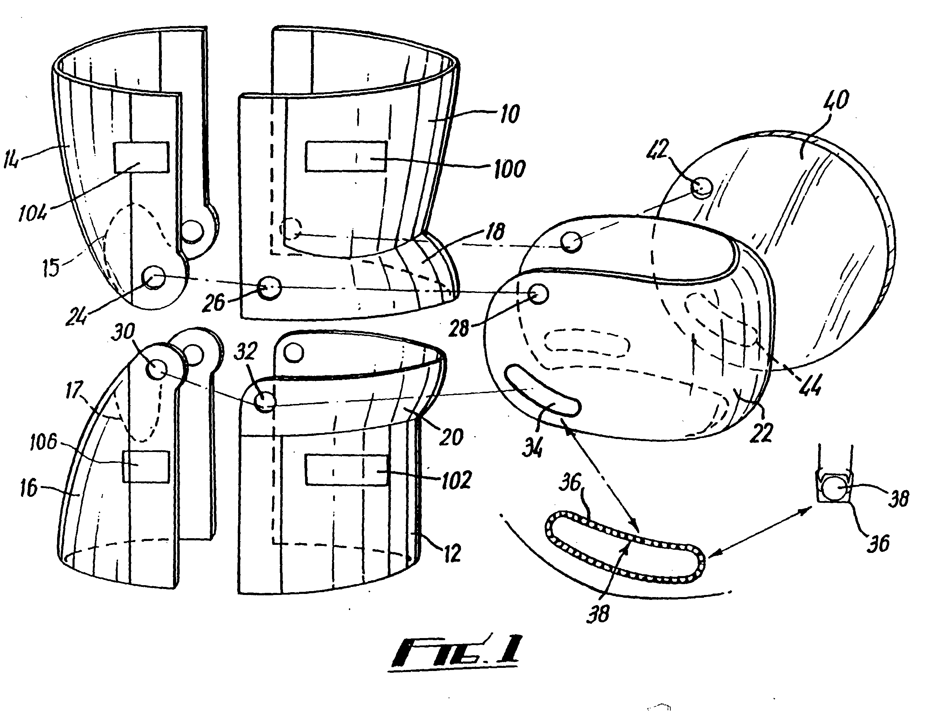

In Figure 1 a knee protector comprises an upper frontal leg support 10, a lower frontal leg support 12, an upper rear leg support 14 and a lower rear leg support 16. Each leg support 10, 12, 14, 16 is curved to fit a human leg (not shown) and conveniently made of rigid but resilient material. Each leg support may be of greater length in the vertical direction than illustrated, to give increased leg support and protection.

The lower part of upper frontal leg support 10 and the upper part of lower frontal leg support 12 are provided with extensions 18, 20 respectively of gradually increasing curvature, or skirt-shaped parts, to accommodate the human kneecap. The gap between the edges of the extensions 18, 20 is covered by a curved knee protector 22 which extends around the upper and lower frontal supports.

The knee protector protects the knee from impacts from the front, and the overall arrangement prevents the knee from being pushed backwards, as the upper and lower supports distribute the impact to the tibia and fibia.

The leg supports 10, 12, 14, 16 and the knee protector 22 are connected together to allow the knee joint to bend in a vertical plane, ie to bend normally.
Conveniently this is achieved by connecting studs (not shown) which pass through apertures in the aforementioned parts. For example, one stud could pass through the circular aperture 24 in upper rear support 14, circular aperture 26 in upper frontal support 10 and circular aperture 28 in the side upper part of knee protector 22. Another stud could pass through circular aperture 30 in lower rear support 16, circular aperture 32 in lower frontal support 12 and kidney-shaped aperture 34 in the side, lower part of knee protector 22.

A similar arrangement of apertures and studs is provided on the side of the protector distant from the direction of viewing.

Such an arrangement will allow normal bending of the knee, with the stud moving along kidney-shaped aperture 34. Preferably this aperture is provided with ball bearings in a retaining channel, as shown by references 36, 38 in the partial enlargement.

There is also provided a circular side impact protector 40, of rigid, resilient material provided with an upper circular aperture 42 and a lower kidney-shaped aperture 44 through which studs can pass as through corresponding apertures in the side of the protector distant from the direction of viewing. The studs would hold the side impact protector 40 in such a position as to protect the knee from side impact.

The lower edge of upper rear support 14 and the upper edge of lower rear support 16 are provided with approximately semi-circular cutouts, 15, 17 indicated by dotted lines, which allows added room for the foot and the leg to pass through the unit, so that the unit can be placed over the knee .

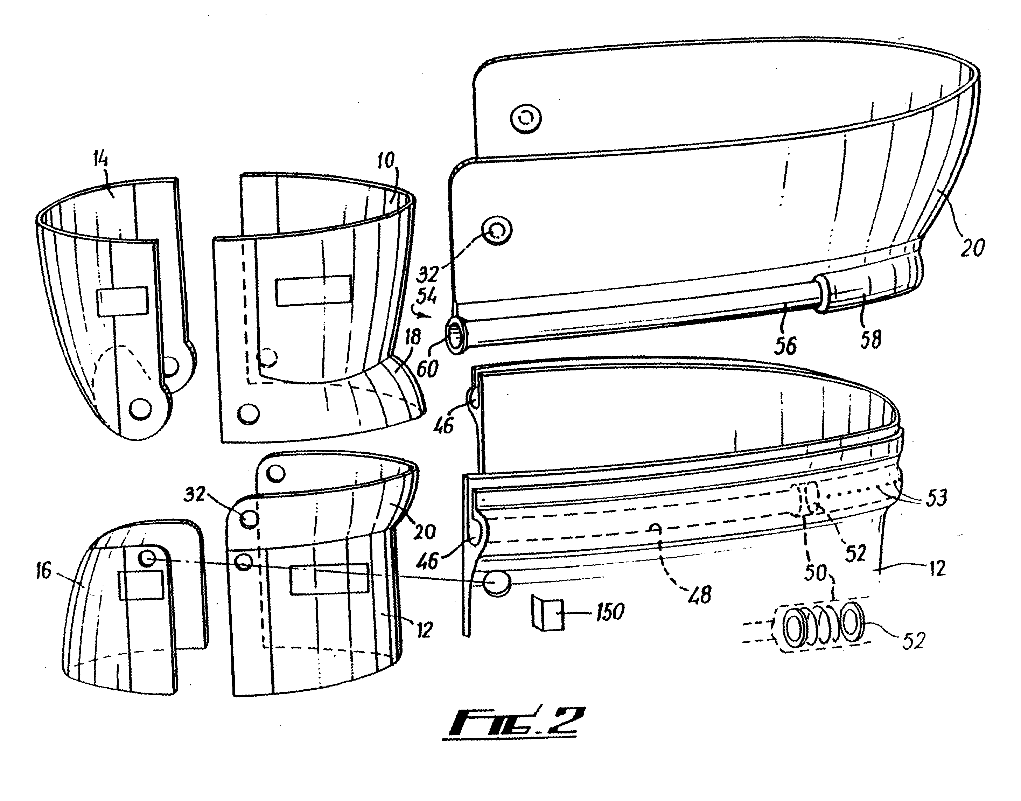

Figure 2 shows upper and lower frontal supports 10, 12 as in Figure 1, and also an enlargement of part of lower support 12, and shows a left leg knee protector, in which the lower support 12 rotates anticlockwise with respect to the upper support 20. The upper support 10 is always held rigid because it is attached to the thigh.

The hinging points to connect the supports 10, 12, 14, and 16 are in a slightly different arrangemant from those in Fig. 1.

The extension 20 is shown-separated from the support 12, to illustrate clearly the predeterminable relative rotation of the two parts.

The upper edge of support 12 is formed as a J-shaped channel 46, of narrow bore 48 from the rear of the support towards the front, and of broader bore 50 around the front and to the rear of the support on the side distant from the viewer.

The change of bore diameter occurs on the protector at a position corresponding to the inside of the human knee within it, and at an angle of about 45° in a horizontal plane from the front of the knee.

Within the broader bore 50 adjacent the change to narrow bore 48 is a spring spacer (washer) 52, shown also in a partial, enlarged view.

The extension 20 carries on its lower edge a J-shaped protrusion 54 having a narrow diameter part 56 and a broader diameter part 58.

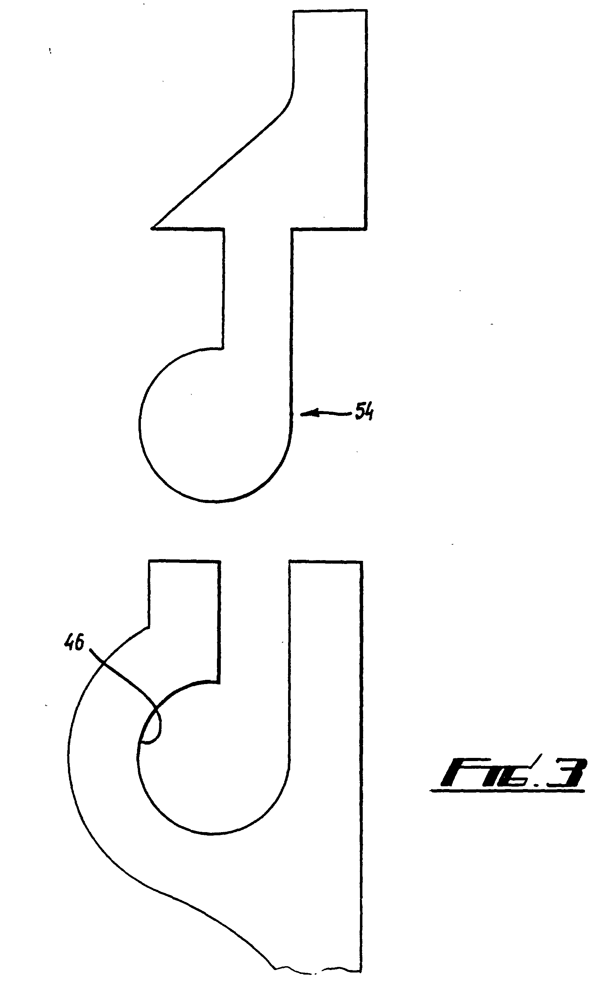

Figure 3 illustrates in section the J-shaped channel 46 and the J-shaped protrusion 54.

Referring once more to Figure 2, the narrow end of 54 is inserted into the broad end of the channel 46 and the extension 20 is rotated clockwise relative to support 12 until it is in the same position in rotation relative to general support 12 as in the Figure. Further rotation is impossible because the broader diameter part 58 of protrusion 54 cannot enter the narrow bore part 48 of channel 46. The narrow diameter part 56 of J-protrusion 54 can however pass through the spring spacer 52, but the spring spacer 52 itself cannot enter the narrow bore channel 48.

When there is human knee within the protector, relative movement of the J-protrusion 54 in J-channel 46 permits the knee to rotate in a substantially horizontal plane. The maximum rotation permitted by the protector will be 45°.

It is sometimes advantageous to limit such rotation, when for example the knee has been injured, or when the natural rotation of the knee is less than 45°. The permitted rotation can be set to the individual requirement of the person wearing the protector by adjusting the spring spacer 52 which can be held in position by placing a screw stud (not shown) through the broad bore part 50 of channel 46. This locks the spacer 52 in position and effectively increases the length of the narrower bore channel 48, and therefore limits rotational movement of J-shaped protrusion 54.

The movement is the same as that of a human leg. If the knee is bent, the lower part of the leg (tibia) can be rotated while the upper part (thigh) is completely immobile. The stop 60 prevents the lower support from moving in the wrong direction, the lower right leg when bent does not rotate anticlockwise, lower left leg when bent does not rotate clockwise.Figure 2 shows a left knee protector, so the lower support 12 rotates anticlockwise in the same direction as the lower leg, and away from the stop 60. The stop 60 prevents movement of the lower support 12 inwards or clockwise, from the central position in which the edges of supports 20 and 12 are in line, as in the Figure.

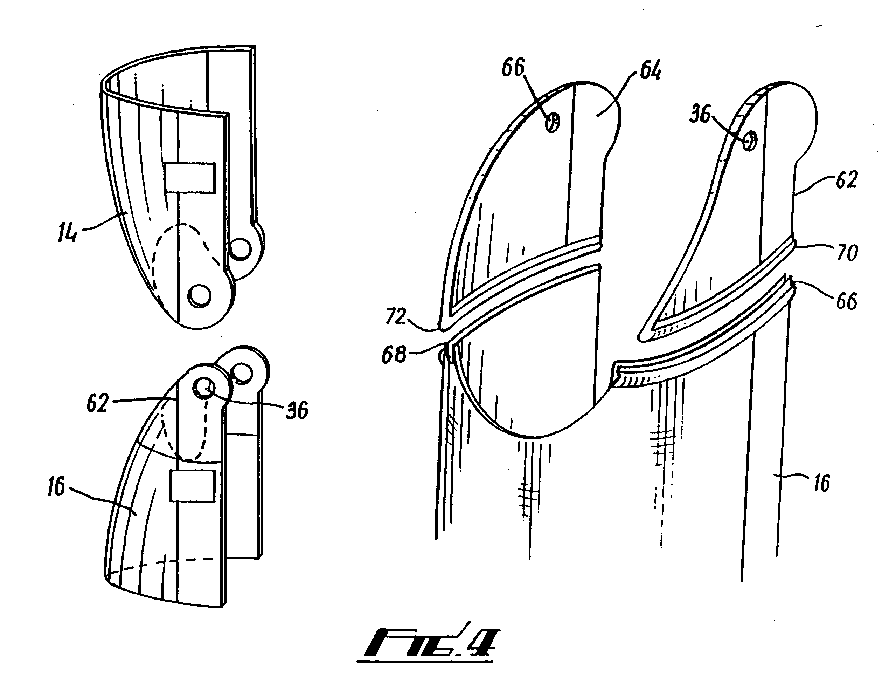

Figure 4 illustrates the arrangement which permits relative rotation of the upper and lower rear supports 14, 16. The lower support 16 has two upper, separable portions 62, 64, one on either side and each having an aperture 36, 66, for a stud (not shown).

The upper edges of the support 16 adjacent the separable portions 62, 64 carry respective J-shaped channels 66, 68 and the lower edges of the separable portions 62, 64 carry J-shaped protrusions 70, 72. The J-protrusions run within the J-channels, as described with reference to Figures 2 and 3.

The limitation of rotation is however predetermined solely by the frontal supports as illustrated in Figure 2.

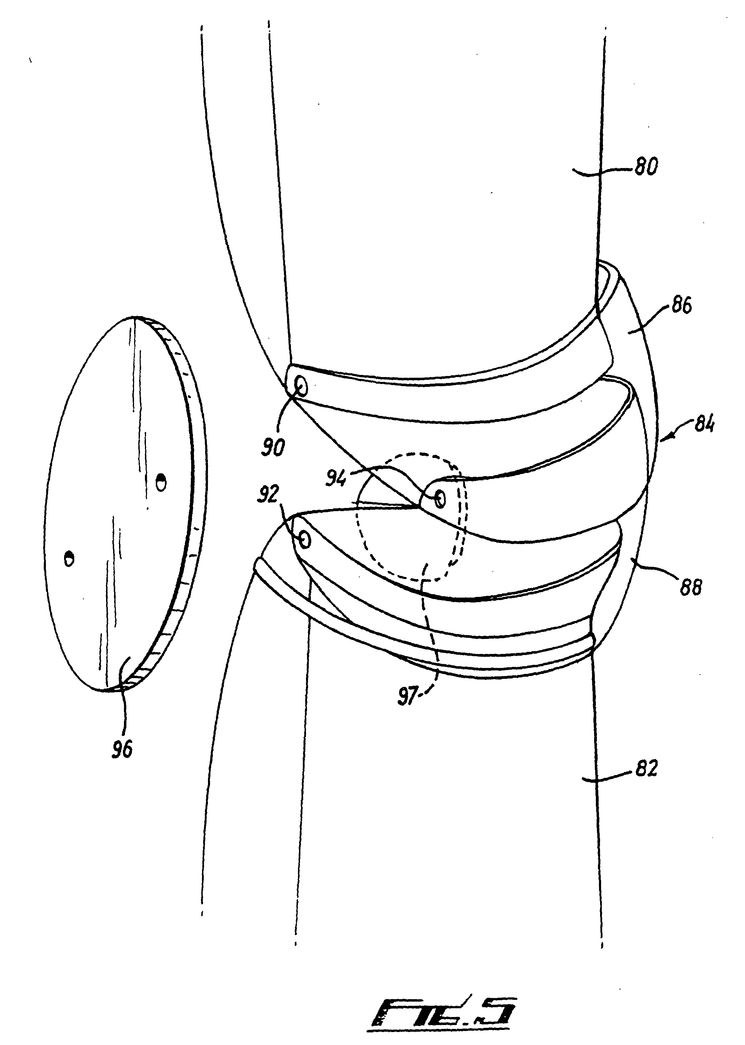

Figure 5 illustrates the frontal supports of a different embodiment of a knee protector having further, optional features.

The protector comprises upper and lower frontal supports 80, 82 and a knee protector indicated generally at 84 which is formed by two overlapping parts 86, 88 hinged at 90, 92 to the upper and lower supports 80, 82 respectively. The two parts of-the knee protector are hinged to each other at 94. Similar hinges are provided at the side of the protector remote from the viewer. The arrangement is such that the overlapping parts 86, 88 overlap by a varying amount in use, limited by the width of the sections 86 and 88. This gives the wearer greater mobility [in bending the knee] than the embodiment shown previously.

A side impact protector, 96, shown spaced from the protector, may be hinged to the knee protector at hinges 94 and 92.

Optionally, there can be placed within the knee protector a spacer 97 (shown largely by the dotted line) of resilient, friction-free material. The spacer can be fixed to the knee protector 94 at such a position that it cushions the anterior cruciate ligament of the knee. The spacer 97 can be of selected thickness, and such a spacer can be provided at each side of the knee to give a very close fit of the knee protector, giving good support of the anterior cruciate ligament.

Such spacers can also be used with the embodiment of Figure 1, and in addition to ligament support can be used to give close fit of a protector to a knee.

It is an advantage of a limb protector according to the invention that it can be individually adjusted to suit the joint it contains. For example, it may be manufactured in a number of basic sizes, which can be adjusted to fit the individual limb within it.

Referring again to Figure 1, the frontal and rear supports may be fastened together by adjustable means, indicated schematically by the rectangles references 100, 102, 104, 106.

The relative spacing of the frontal and rear supports can be individually varied to give a good fit.

Preferably the adjustable means as such that the adjustment can be preset, and subsequent removal and replacement of the protector can be relatively rapid and simple.

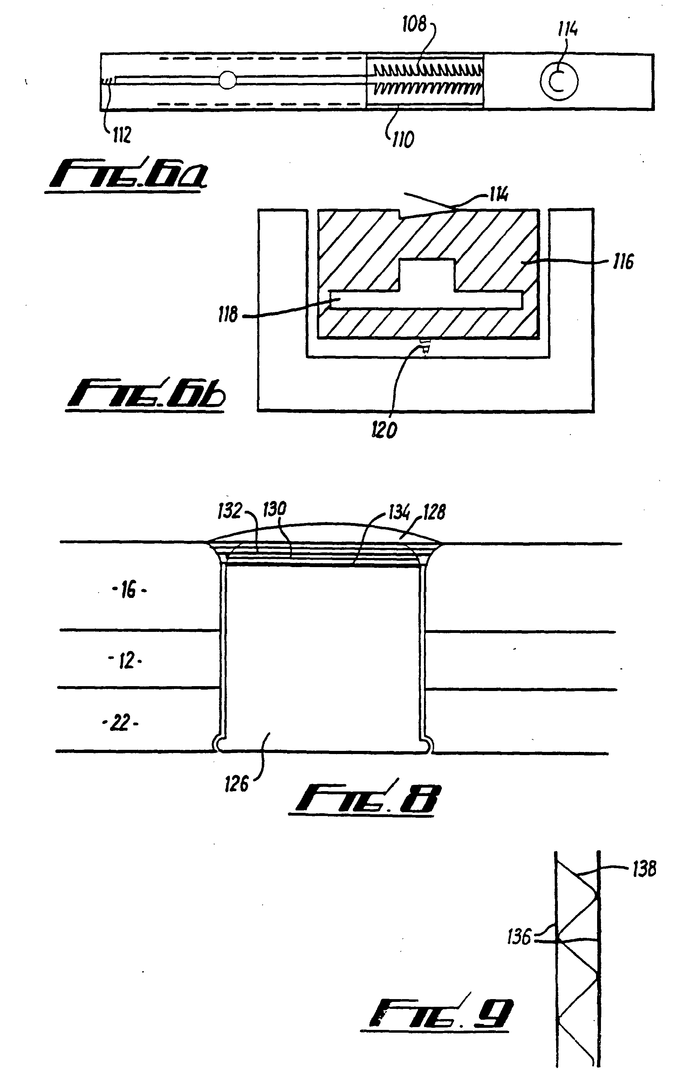

An example of such an adjustable means is shown in Figure 6. Figure 6(a) shows a ratchet 108 within a cover 110 which is spring loaded to prevent exposure of the ratchet and which retreats as the ratchet moves. There is a locking mechanism with a ring pull 114.

Figure 6(b) shows an end view of Figure 6(a). Below the ring pull 114 there is a piston type mechanism 116 having an inverted T-shaped gap 118; when the ring pull is pulled, the gap 118 allows the ratchet 108 to run free within the cover 110. As spring retainer 120 returns the smaller gap of the inverted T-shape, which is shaped like a sawtooth so that when it is released, fits perfectly between the ratchet teeth, prevents the ratchet from moving back and forth, thus lacking the ratchet in place.

The ratchet can therefore be adjusted to give the required separation of the frontal and rear supports for an individual leg. A torsion spring 112 then allows minimal expansion of the ratchet, to correspond to muscular expansion of the leg.

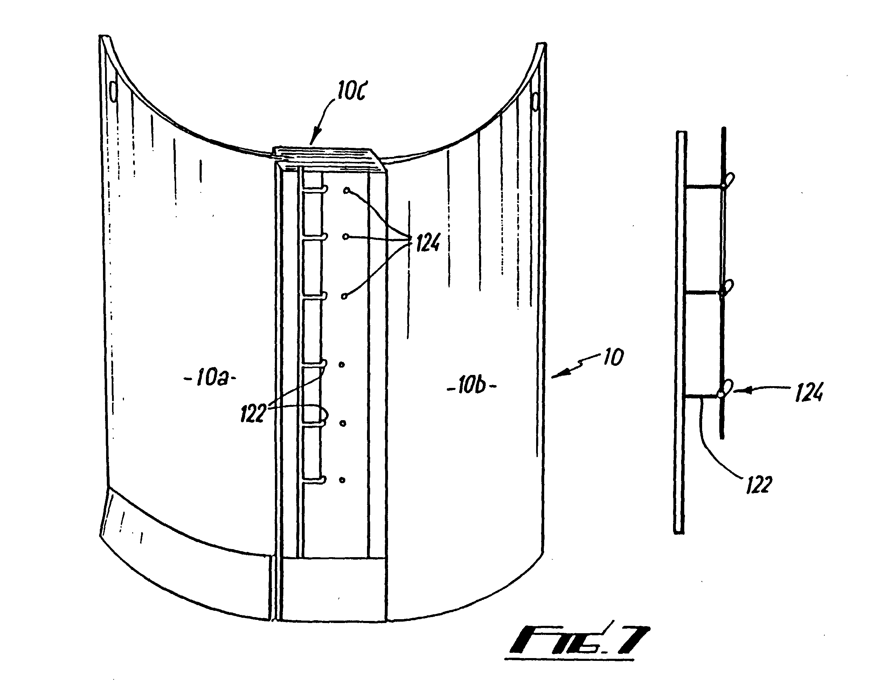

In Figure 7, a method of adjusting the size of a knee protector is illustrated. The upper frontal support 10 is formed in two parts 10(a) and 10(b) with a vertical overlap 10(c). The overlap on part 10(b) has two series of vertically-separated perforations, 124. The overlap on part 10(a) has a series of projecting studs 122 at the same distance as the perforations, and having right-angle ends which can enter the perforations. The amount of overlap is therefore adjustable, eg by 2 cm, and the size of the support 10 can be selected as appropriate.

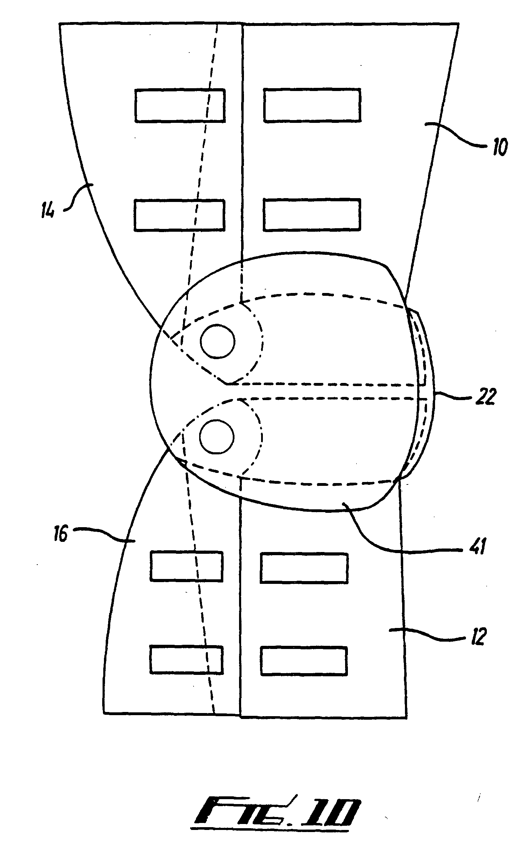

Figure 8 illustrates one of the studs which hinge the upper and lower, frontal and rear supports together. The stud 126 passes through the thickness of; for example, the lower rear support 16, lower frontal support 12, and knee protector 22. The head of the stud is protected by a cover cap 128 which is bullet-shaped to allow easy access of a split pin which is sandwiched between a spring washer 132 and a plain washer 134. The cover cap 128 is screwed into the top of the stud 126 and is prevented from coming undone by the spring washer 132.

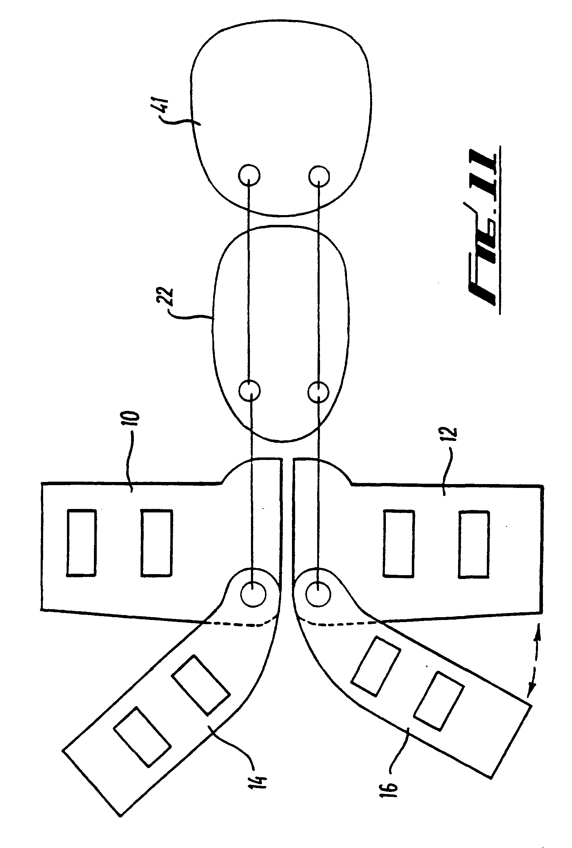

Figure 9 shows that any of the supports or knee protector can be made of a sandwich construction 136. Either in the inside of the sandwich contains a corrugated impact-absorbing material 138, or some other material is used that has relevant absorbtion coefficients.

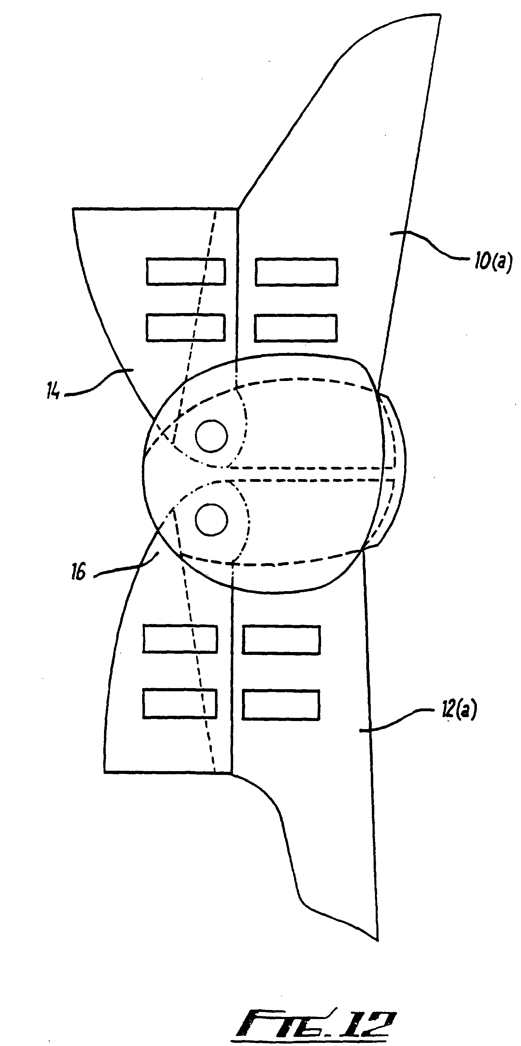

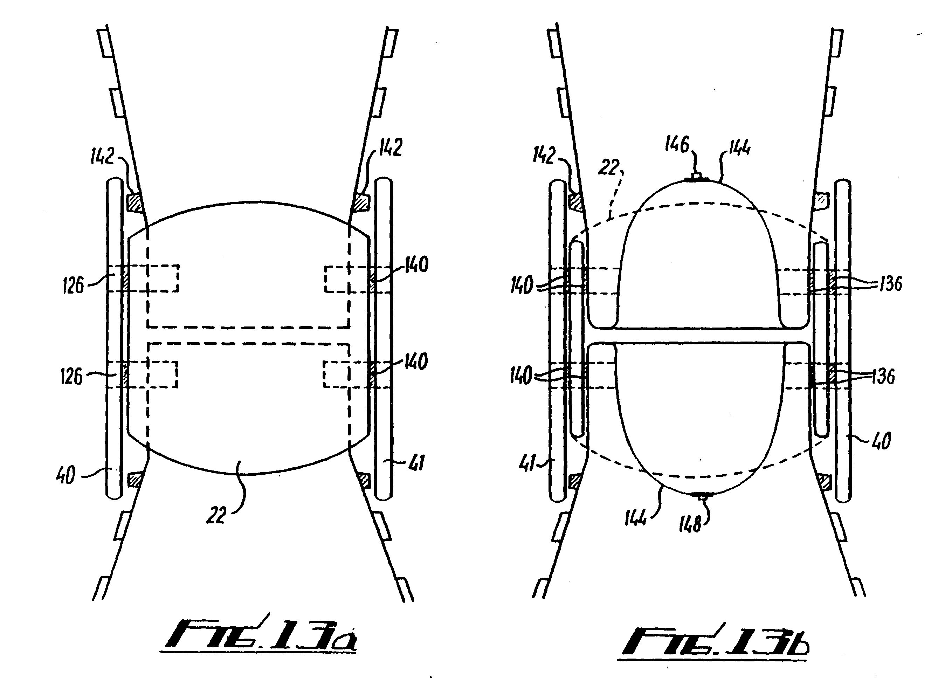

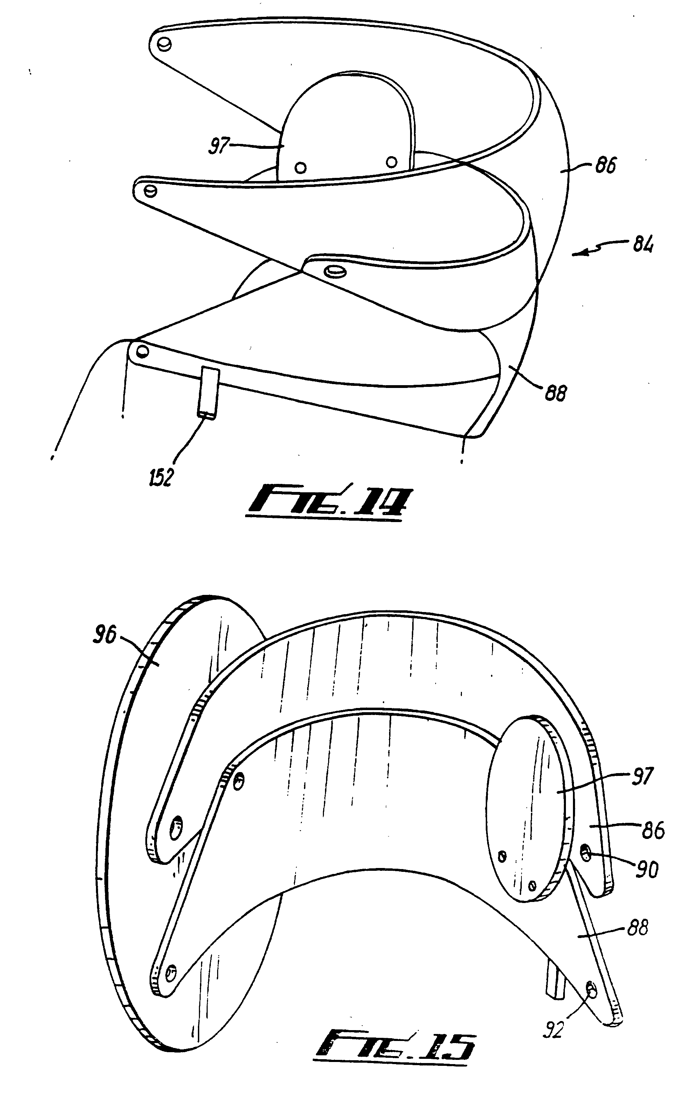

Figure 10 shows in assembled form a knee protector as illustrated in Figures 1 and 2. Supports 10, 12, 14, 16 and knee protector 22 are shown in their operative positions, together with side impact protector 41. Figure 11 shows how the same parts can be hinged to allow a wearer to place it on a knee, or remove it. Figure 12 shows a variation of a knee protector in which the upper and lower frontal supports 10(a), 12(a) are extended to give improved protection to the thigh and shin.

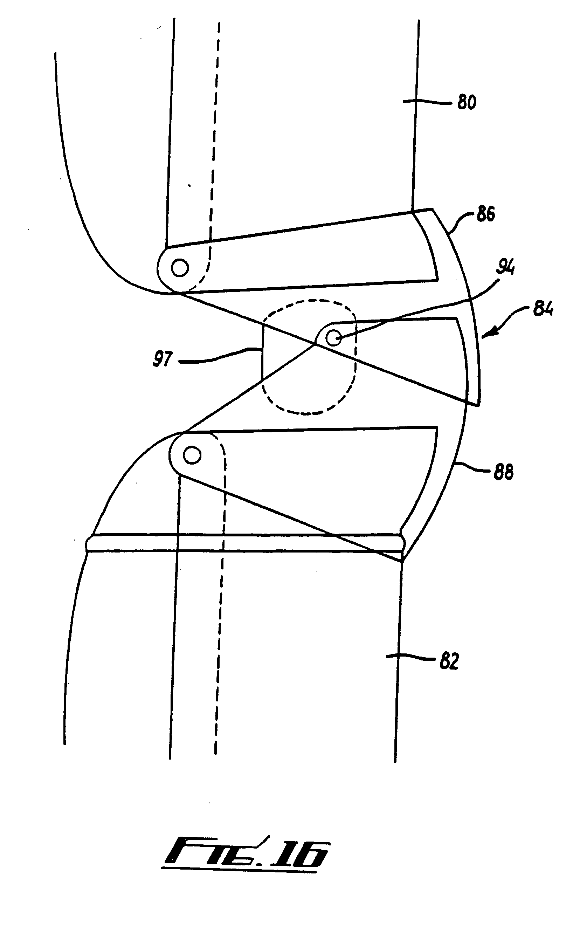

In Figure 13, 13(a) is a view from the front of a knee protector and 13 (b) is a view from the rear of a knee protector. In Figure 13(a), the side impact protectors 40, 41 are connected to the knee protector 22 and other parts of the protector by studs 126. As will be seen, there are spacers 140 between the knee protector 22 and the side impact protectors 40, 41. There are also absorption pads 142 to take pressure from the side impact protectors 40, 41 when force is applied to them, to help distribute the impact force throughout the whole protector unit.

Figure 13(b) also shows cutaway sections on the upper and lower rear supports which allow the wearers foot to pass through when the rear supports are hinged open as shown in Figure 11.

Figure 13(b) also illustrates the attachment points 146, 148 for an adjustable padded torsion bar (not shown) which can be locked to the protector behind the knee. This allows the protector to be used as a light weight temporary splint.

A joint protector according to the invention allows free hinging of a joint such as a knee in the normal way, and predetermined rotational movement. In some circumstances however it may be advantageous to prevent rotational movement.

Referring again to Figure 2, the main part of lower frontal support 12 carries an L-shaped projection 150 the female part of a coupling.

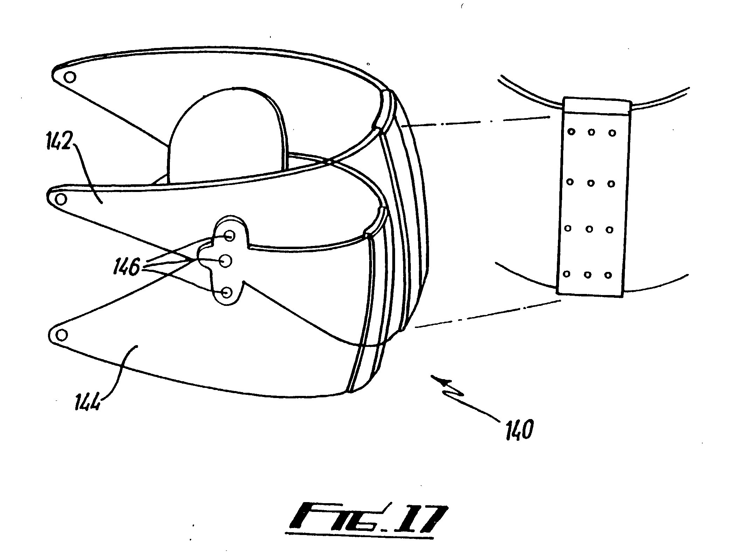

Figure 14 shows a two-part knee protector as in Figure 5. Part 88 carries a male part of a coupling, 152 in the form of a square section rod.- If the two parts of the coupling fit together, rotation of the protector is prevented. The two parts will be brought into this position when the wearer of the knee protector stands with a straight leg, and will uncouple when the wearer bends the knee. The lock thus works in a parallel way to the human leg.

Figure 15 is a view of a two-part knee protector from inside, showing the two parts 86, 88, the hinge-points 90, 92, removable spacer 97 to protect the anterior cruciate ligament, and side impact protector 96 with its hinge points.

Figure 16 is a schematic view of the protector of Figure 5, illustrating the hinge unit 94 which holds the two parts 86, 88, of the knee protector together.

Figure 17 illustrates a variation of the knee protector shown in Figures 14 and 15, with the size adjustment device shown in Figure 7. The knee protector 140 has two overlapping part 142, 144, hinged together at hinge point 146 which has 3 hinge positions to allow adjustment to fit an individual knee.

While the parts of the knee protector have been referred to as rigid, it is preferable to use a resilient material which by nature has impact-absorbing properties. Such a material is carbon fibre composite material.

The spacers 97 are conveniently made of a softer material with relevant absorption coefficients, such as polypropylene.

The inside of the knee protector may be line with a padding of stretch, anti-friction material, which may also extend to the insides of the upper and lower frontal supports. Such a lining allows the protector to move smoothly over the knee and helps to prevent pinching or damage to the kneecap.

The invention has been described with reference to a knee protector, it is also applicable to an elbow protector.

## Claims

1. A limb protector comprising an upper rigid curved support (10); a lower rigid curved support (12); and means (46, 54) for permitting relative movement of the upper support and lower support, characterised in that said relative movement is rotational movement in a plane substantially parallel to the planes of curvature of the upper and lower curved supports.

2. A limb protector according to Claim 1 in which the amount of relative rotation is adjustable.

3. A limb protector according to Claim 1 further comprising means (14, 16, 100, 102, 104, 106) to attach the upper (10) and lower (12) curved supports to a limb above and below a joint.

4. A limb protector according to Claim 1 further comprising means (24, 26, 30, 32) for permitting relative hinging of the upper (10) and lower (12) supports in a plane orthogonal to the plane of relative rotation.

5. A limb protector according to Claim 1 in which one of the upper (10) and lower (12) supports is provided with an extension (20) to render one of the supports in two-part form (12, 20) with a sliding joint (46, 54) between the two parts, the extension (20) being attached to the other support (10) so as to permit relative rotation of the upper and lower supports.

6. A limb protector according to Claim 5 in which said sliding joint (46, 54) is provided in the lower support (12) adjacent to the upper support (10).

7. A limb protector according to Claim 6 for protecting a leg in which the sliding joint (46, 54) is arranged in the lower support so that relative rotation occurs immediately below the kneecap.

8. A limb protector according to Claim 4 in which there is further provided locking means (50, 52) to prevent relative rotary movement of the upper (10) and lower (12) supports on extension of the limb.

9. A limb protector according to Claim 8 in which the locking means comprises male and female parts (50, 52) which cooperate when the limb is fully straightened.

10. A limb protector according to Claim 4 further comprising a hinge-covering means (22) which overlaps the adjacent edges of the upper and lower supports (10, 12) during a hinging movement.

11. A limb protector according to Claim 10 in which the hinge-covering means (84) is provided in two parts (86, 88) one connected pivotally to each of the upper and lower supports (80, 82) and further connected pivotally to each other.

12. A limb protector according to Claim 11 for protecting a leg in which the two parts (86, 88) of the hinge-covering means (84) are pivotally connected (90, 92) respectively to the upper and lower supports (80, 82) at the side of the leg and the parts of the hinge-covering means are pivotally connected to each other (94) at the side of the leg forward of the pivotal connection (90, 92) to the supports (80, 82).

13. A limb protector according to Claim 12 in which, when the limb is flexed, the hinging axis of the upper and lower supports (80, 82) move downwards and backwards and when the limb is extended, the hinging axis moves upwards and forwards.

14. A limb protector according to Claim 1 in which the upper support comprises a curved frontal support (10) and a curved rear support (14) connected to each other by hinge means (24, 26), and the lower support comprises a curved frontal support (12) and a curved rear support (16) connected to each other by hinge means (30, 32).

15. A limb protector according to Claim 14 further comprising hinge-covering means (22) hinged to the upper and lower frontal supports (10, 12).

16. A limb protector according to Claim 1 further comprising a side impact protector (40) exterior to and partially overlapping each of the upper and lower supports (10, 12).

17. A limb protector according to Claim 1 further comprising spacer means (97) within and partially overlapping each of the upper and lower supports (80, 82), the spacer means (97) being of such a thickness that the limb protector is a close fit on a limb within it.

18. A limb protector according to Claim 1 further comprising means (122, 124) to adjust the width and adjust the depth of the protector.

## Patentansprüche

1. Ein Gliedschutz bestehend aus einer oberen starren gekrümmten Stütze (10), einer unteren starren gekrümmten Stütze (12) und das Mittel (46, 54) zur Ermöglichung der relativen Bewegung der oberen und unteren Stütze, so geschaffen, daß die besagte relative Bewegung eine Drehbewegung in einer Ebene darstellt, die im wesentlichen parallel zur Krümmung der oberen und unteren gekrümmten Stützen verläuft.

2. Ein Gliedschutz gemäß Behauptung 1, bei welchem sich das Ausmaß der relativen Drehbewegung einstellen läßt.

3. Ein Gliedschutz gemäß Behauptung 1, der außerdem über Mittel (14, 16, 100, 102, 104, 106) zur Anbringung der oberen (10) und unteren (12) gekrümmten Stützen an ein Gliedmaß oberhalb und unterhalb eines Gelenks verfügt.

4. Ein Gliedschutz gemäß Behauptung 1, der außerdem über Mittel (24, 26, 30, 32) zur Ermöglichung der relativen Befestigung der oberen (10) und unteren (12) Stützen in einer Ebene senkrecht zur Ebene der relativen Drehbewegung verfügt.

5. Ein Gliedschutz gemäß Behauptung 1, in welchem eine der oberen (10) und unteren (12) Stützen mit einer Verlängerung (20) versehen ist, um eine der Stützen in zweiteiliger Form (12, 20) mit einem Schiebegelenk (46, 54) zwischen den beiden Teilen anzubieten, wobei die Verlängerung (20) an der anderen Stütze (10) angebracht wird, so daß die relative Drehbewegung der oberen und unteren Stützen ermöglicht wird.

6. Ein Gliedschutz gemäß Behauptung 5, in welchem das genannte Schiebegelenk (46, 54) in der unteren Stütze (12) neben der oberen Stütze (10) angebracht wird.

7. Ein Gliedschutz gemäß Behauptung 6 zum Schutz eines Beins, bei welchem das Schiebegelenk (46, 54) so in der unteren Stütze angebracht wird, daß die relative Drehbewegung unmittelbar unterhalb der Kniekappe erfolgt.

8. Ein Gliedschutz gemäß Behauptung 4, welcher weitere Verschlußmittel (50, 52) aufweist, um die relative Drehbewegung der oberen (10) und unteren (12) Stützen beim Ausstrecken des Gliedmaßes zu verhindern.

9. Ein Gliedschutz gemäß Behauptung 8, dessen Verschlußmechanismus aus einer Buchse und einem Steckstück (50, 52) besteht, welche bei voller Ausdehnung des Gliedmaßes einrasten.

10. Ein Gliedschutz gemäß Behauptung 4, der außerdem über eine Scharnierabdeckung (22) verfügt, welche die nebeneinanderligenden Enden der oberen und unteren Stützen (10, 12) während einer Scharnierbewegung überlappt.

11. Ein Gliedschutz gemäß Behauptung 10, dessen Scharnierabdeckung (84) in zwei Teilen (86, 88) erstellt wird, wobei einer mittels eines Drehpunkts an jede der oberen und unteren Stützen (80, 82) verbunden wird und zudem beide mittels eines Drehpunkts miteinander verbunden werden.

12. Ein Gliedschutz gemäß Behauptung 11 zum Schutz eines Beins, bei welchem die beiden Teile (86, 88) der Scharnierabdeckung (84) mittels Drehpunkten (90, 92) jeweils mit den oberen und unteren Stützen (80, 82) an der Seite des Beins verbunden werden, und die Teile der Scharnierabdeckung mittels Drehpunkt miteinander (94) an der Seite des Beins vor der Drehpunktverbindung (90, 92) zu den Stützen (80, 82) verbunden werden.

13. Ein Gliedschutz gemäß Behauptung 12, bei welchem bei der Beugung des Knies die Scharnierachsen der oberen und unteren Stützen (80, 82) sich nach unten und hinten bewegen und sich bei der Ausdehnung des Gliedmaßes die Scharnierachsen nach oben und vorne bewegen.

14. Ein Gliedschutz gemäß Behauptung 1, bei welchem die obere Stütze aus einer gekrümmten vorderen Stütze (10) und einer gekrümmten hinteren Stütze (14) besteht, welche miteinander durch Scharniere (24, 26) verbunden sind, und die untere Stütze aus einer gekrümmten vorderen Stütze (12) und einer gekrümmten hinteren Stütze (16) besteht, welche miteinander durch Scharniere (30, 32) verbunden sind.

15. Ein Gliedschutz gemäß Behauptung 14, der außerdem über eine Schamierabdeckung (22) verfügt, die an den oberen und unteren vorderen Stützen (10, 12) aufgehängt wird.

16. Ein Gliedschutz gemäß Behauptung 1, der außerdem über einen Seitenstoßschutz (40) außerhalb von und teilweise mit den oberen und unteren Stützen (10, 12) überlappend verfügt.

17. Ein Gliedschutz gemäß Behauptung 1, der außerdem über Abstandsstück (97) innerhalb der oberen und unteren Stützen (80, 82) und diese teilweise überlappend verfügt, wobei diesen Abstandsstück von einer Stärke ist, der für eine dichte Anpassung des Gliedschutzes an das darin enthaltene Gliedmaß sorgt.

18. Ein Gliedschutz gemäß Behauptung 1, der außerdem über Mittel (122, 124) zur Einstellung der Breite und Tiefe des Schutzes verfügt.

## Revendications

1. Un protecteur de membre comprenant un support courbé rigide supérieur (10) ; un support courbé rigide inférieur (12) ; et des moyens (46, 54) permettant un mouvement relatif du support supérieur et du support inférieur, caractérisé dans ce dit mouvement relatif est un mouvement rotatoire dans un plan substantiellement parallèles aux plans de courbature des supports courbés supérieurs et inférieurs.

2. Un protecteur de membre selon la Revendication 1 dans laquelle la quantité de rotation relative est ajustable.

3. Un protecteur de membre selon la Revendication 1 comprenant en plus des moyens (14, 16, 100, 102, 104, 106) pour attacher les supports courbés supérieurs (10) et inférieurs (12) au membre au-dessus et en dessous d'une articulation.

4. Un protecteur de membre selon la Revendication 1 comprenant en plus des moyens (24, 26, 30, 32) pour permettre une articulation relative des supports supérieurs (10) et inférieurs (12) dans un plan orthogonal au plan de rotation relative.

5. Un protecteur de membre selon la Revendication 1 dans laquelle l'un des supports supérieurs (10) et inférieurs (12) est fourni avec une extension (20) pour rendre l'un des supports dans une forme à deux parties (12, 20) avec une articulation coulissante (46, 54) entre les deux parties, l'extension (20) étant attachée sur l'autre support (10) pour permettre une rotation relative des supports supérieur et inférieur.

6. Un protecteur de membre selon la Revendication 5 dans laquelle la-dite articulation coulissante (46, 54) est fournie dans le support inférieur (12) sadjacent au support supérieur (10).

7. Un protecteur de membre selon la Revendication 6 pour protéger une jambe dans laquelle une articulation coulissante (46, 54) est arrangée dans le support inférieur pour qu'une rotation relative se produise immédiatement sous la rotule.

8. Un protecteur de membre selon la Revendication 4 dans laquelle il y a d'autres moyens de verrouillage (50, 52) pour empêcher un mouvement rotatoire relatif des supports supérieurs (10) et inférieurs (12) sur l'extension du membre.

9. Un protecteur de membre selon la Revendication 8 dans laquelle les moyens de verrouillage comprennent des pièces mâles et femelles (50, 52) qui coopèrent lorsque le membre est entièrement redressé.

10. Un protecteur de membre selon la Revendication 4 comprend en plus un moyen de couverture d'articulation (22) qui se superpose sur les bords adjacents des supports supérieur et inférieur (10, 12) lors d'un mouvement d'articulation.

11. Un protecteur de membre selon la Revendication 10 dans laquelle le moyen de couverture d'articulation (84) est fourni en deux parties (86, 88), une reliée de façon pivotante à chacun des supports supérieur et inférieur (80, 82) et également reliée de façon pivotante les unes aux autres.

12. Un protecteur de membre selon la Revendication 11 pour protéger une jambe dans laquelle les deux parties (86, 88) du moyen de couverture d'articulation (84) sont reliées de façon pivotante (90, 92) respectivement aux supports supérieur et inférieur (80, 82) sur le côté de la jambe et les parties du moyen de couverture d'articulation sont reliées de façon pivotante les unes aux autres (94) sur le côté de la jambe vers l'avant de la connexion à pivot (90, 92) aux supports (80, 82).

13. Un protecteur de membre selon la Revendication 12 dans laquelle, lorsque le membre est plié, l'axe d'articulation des supports supérieur et inférieur (80, 82) descend et recule et lorsque le membre est tendu, l'axe d'articulation monte et avance.

14. Un protecteur de membre selon la Revendication 1 dans laquelle le support supérieur comprend un support frontal courbé (10) et un support arrière courbé (14) reliés l'un à l'autre par un moyen d'articulation (24, 26), et le support inférieur comprend un support frontal courbé (12) et un support arrière courbé (16) reliés l'un à l'autre par un moyen d'articulation (30, 32).

15. Un protecteur de membre selon la Revendication 14 comprend en plus un moyen de couverture d'articulation (22) articulé aux supports frontaux supérieurs et inférieurs (10, 12).

16. Un protecteur de membre selon la Revendication 1 comprend en plus un protecteur d'impact latéral (40) extérieurs à chacun des supports supérieur et inférieur (10, 12) et en les superposant partiellement.

17. Un protecteur de membre selon la Revendication 1 comprend en plus un moyen d'écartement (97) intérieurs à chacun des supports supérieur et inférieur (80, 82) et en les superposant partiellement, le moyen d'écartement (97) étant d'une telle épaisseur que le protecteur de membre est ajusté étroitement sur le membre à l'intérieur.

18. Un protecteur de membre selon la Revendication 1 comprend en plus un moyen (122, 124) pour ajuster la largeur et pour ajuster la profondeur du protecteur.
